# DEMANDE DE BREVET EUROPEEN

(11) **EP 1 156 317 A2**
(43) Date de publication de la demande: **21.11.2001**
(21) Numéro de dépôt: 01401250.4
(22) Date de dépôt: 15.05.2001
(51) Int. Cl.: G01N 3/40, G01N 33/24, E02D 1/02

(54) **Kit de pénétrométrie**

(30) Priorité: 15.05.2000 FR 0006145
(71) Demandeur: EMCI, 78190 Trappes (FR)
(72) Inventeur: Piau, Christophe, 91150 Etampes (FR)
(74) Mandataire: Debay, Yves

(57) **Abrégé**

La présente invention concerne un kit de pénétrométrie (1) constitué d'une tige d'enfoncement (11) surmontée d'une enclume (12) sur laquelle vient taper en chute libre une masse (14) caractérisé en ce qu'au centre de la masse (14), sur toute son épaisseur est réalisé un perçage (141) par lequel passe une tige (13) rendue solidaire de l'enclume (12) qui se dresse perpendiculairement au plan constitué par la face supérieure de l'enclume (12), un équipage mobile (15) à tiroir hydraulique se déplace alternativement sur ladite tige (13) entre une position haute et une position basse, l'équipage mobile (15) comportant des moyens de libération de la masse (14) arrivée en position haute pour permettre sa chute libre et des moyens de solidarisation avec la masse (14) lors de son arrivée en position basse, un piston-tiroir (152) actionné par ces moyens de solidarisation et de libération inversant le sens de déplacement de l'équipage mobile (15)

## Description

La présente invention concerne un kit de pénétrométrie, c'est à dire un dispositif pour soulever une masse de masse x kg à une hauteur y et de la relâcher de manière automatisée et cyclique sur une tige dont on mesure la pénétration dans le sol. Un pénétromètre doit soulever une masse toujours identique à des hauteurs de chute identiques et de manière répétitive et la relâcher afin de provoquer l'enfoncement de la tige dans le sol. Ces mesures de pénétration ainsi effectuées permettent de décrire les caractéristiques mécaniques du sol.

Les appareils actuels, pour la plupart, soulèvent la masse grâce à un câble ou une chaîne et la relâchent par des moyens d'embrayage divers, donnant des zones de frottement entraînant des pertes sur la masse. De plus la masse est tirée par sa face supérieure et est guidée par deux glissières latérales, ce qui entraîne des frottements supplémentaires.

Pour disposer d'une hauteur de chute toujours identique, ces dispositifs sont dotés de capteurs, mais ces capteurs ont des difficultés à suivre l'évolution et les variations d'enfoncement de la tige suivant le terrain et donc de la masse qui vient taper dessus.

Les appareils existants sont aussi généralement dédiés et souvent très lourds, obligeant les utilisateurs à mobiliser d'importants moyens de manutention.

La présente invention a donc pour objet de pallier les inconvénients de l'art antérieur en proposant un kit permettant de relâcher la masse à une hauteur toujours identique par rapport à la tige pénétrante avec des frottements minimes sur la masse. De plus, le dispositif selon l'invention permet d'avoir un matériel léger et facilement transportable.

Ce but est atteint selon la revendication 1. Des développements supplémentaires sont décrits dans les revendications 2 à 20.

L'invention, avec ses caractéristiques et avantages, ressortira plus clairement à la lecture de la description faite en référence aux dessins annexés dans lesquels :
- les figures 1, 2, 3 et 4 représentent le kit de pénétrométrie en coupe dans différentes phases de son fonctionnement.
- La figure 5 représente une vue en perspective de l'équipage mobile à tiroir hydraulique.
- La figure 6 représente une vue en coupe de l'équipage mobile à tiroir hydraulique en prise de masse.
- La figure 7 représente l'équipage mobile à tiroir hydraulique vu sous deux coupes différentes.
- La figure 8 représente le principe de base d'alimentation d'un vérin à travers l'équipage mobile à tiroir hydraulique.

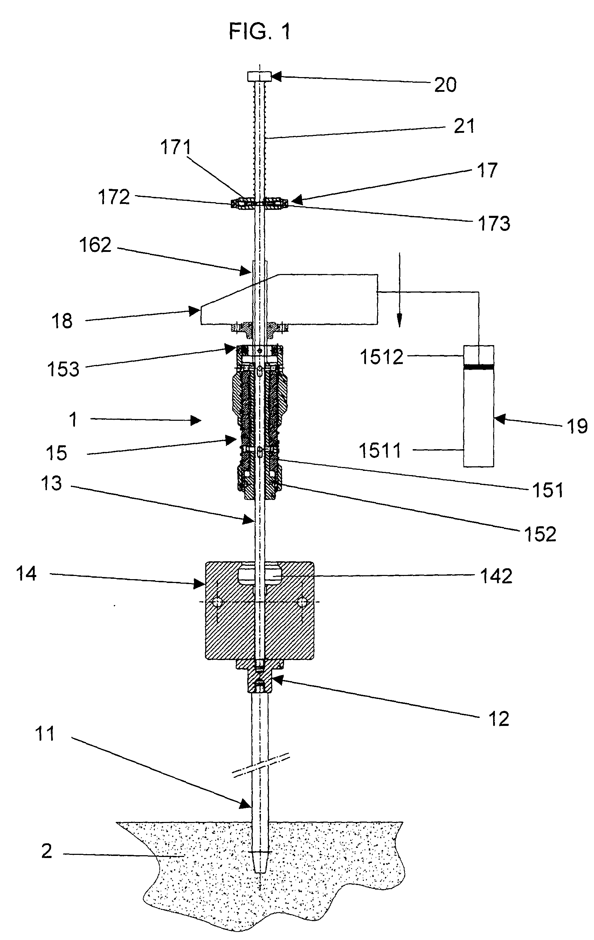

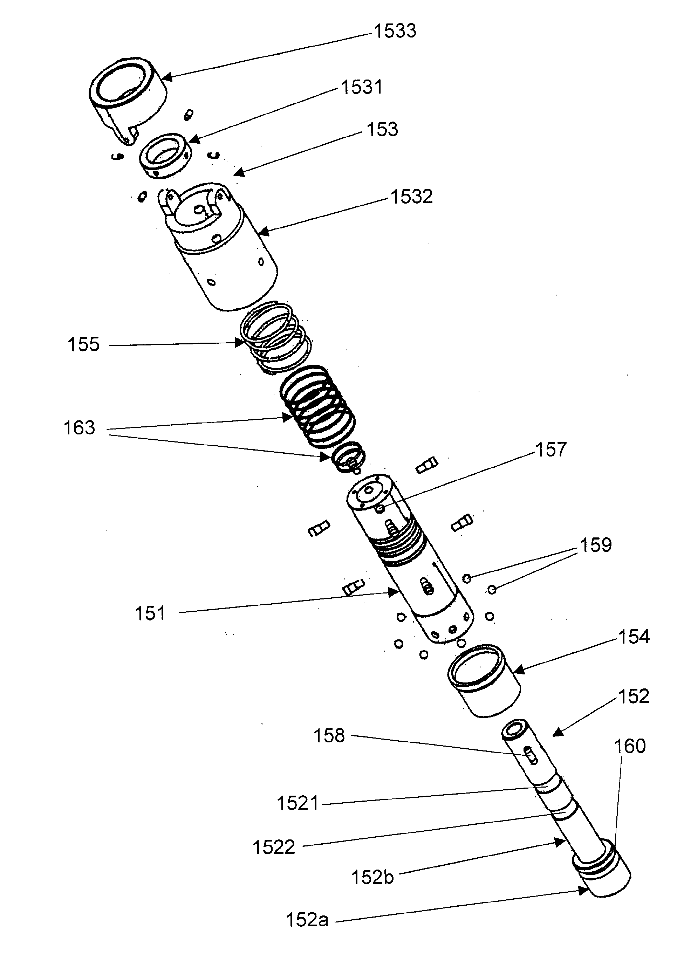

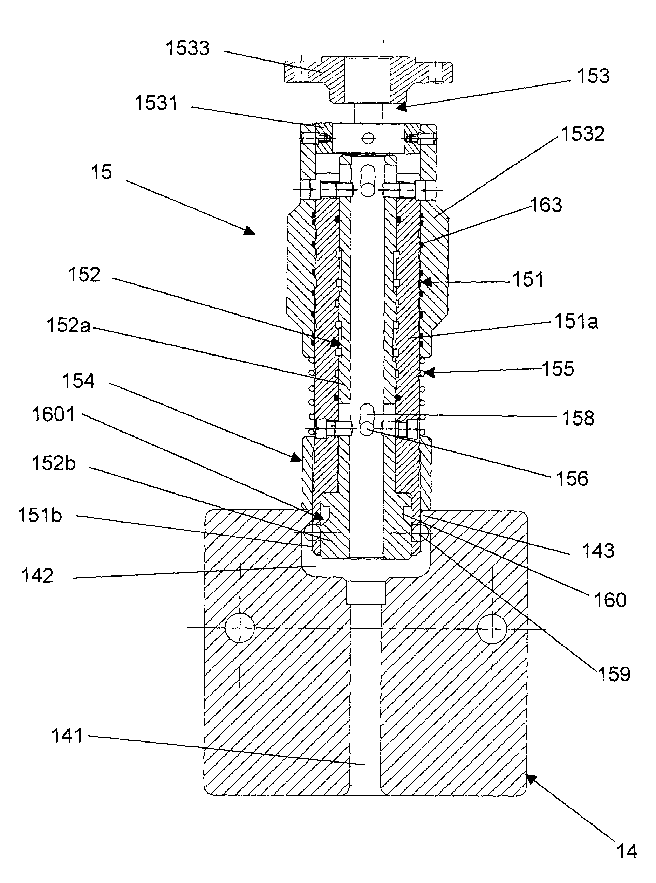

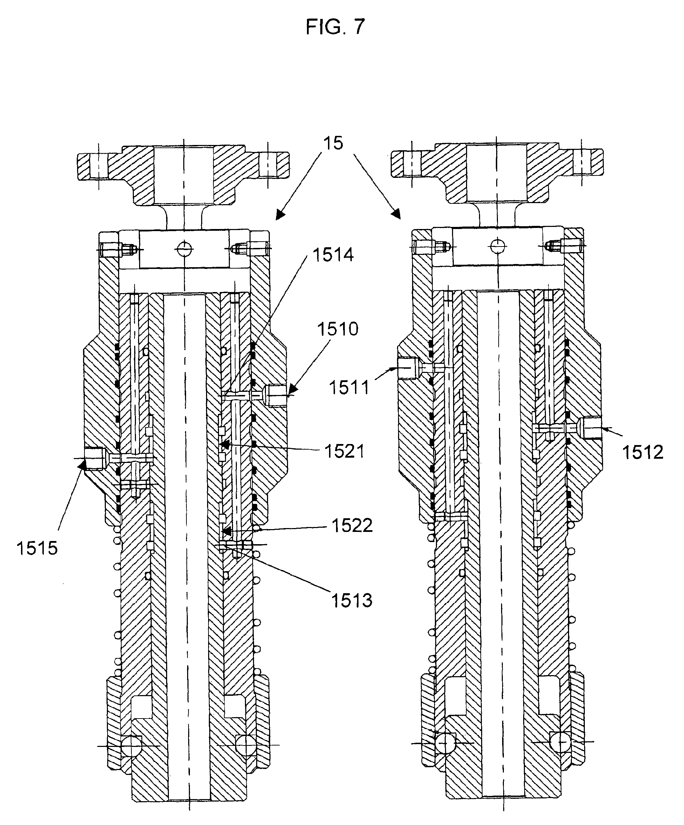

L'invention sera décrite en liaison des figures 1 à 8.

Le kit de pénétrométrie (1) selon l'invention est constitué d'une tige d'enfoncement (11) destinée à s'enfoncer perpendiculairement dans le sol (2) et vissée par son extrémité supérieure dans la face inférieure d'une enclume (12. Une tige (13) vissée sur la face supérieure de l'enclume (12) se dresse perpendiculairement au plan constitué par la face supérieure de cette enclume (12). Une masse (14) coulisse sur la tige (13) en étant guidée par l'intermédiaire d'un perçage (141) formé sur toute l'épaisseur de la masse (14) en son centre, pour venir en chute libre taper sur l'enclume (12) lorsqu'elle est lâchée d'une certaine hauteur par un équipage mobile (15) à tiroir hydraulique susceptible de se déplacer le long de la tige (13) entre la masse (14) et une butée (17) fixée sur la tige (13) au-dessus de lui. L'équipage mobile (15) coulisse grâce à un chariot (18) mobile sur une potence parallèle à la tige (13) et actionné par un moteur ou un vérin (19). En règle générale la masse (14) pèse 64 kg et est lâchée d'une hauteur de 750 mm. La tige (13) est graduée de manière à pouvoir faire varier la hauteur de chute.

L'équipage mobile (15) à tiroir hydraulique selon l'invention comprend principalement un corps (151), un piston-tiroir (152), une bague (154) et un ressort (155). Il est entraîné dans ses phases de montée ou de descente le long de la tige (13) par un moteur ou un vérin (19) dont la direction est asservie au déplacement du tiroir hydraulique.

Le corps (151) est creux, a une forme extérieure cylindrique et un espace intérieur ayant la forme de deux cylindres (151a, 151b) fourreau de diamètres différents accolés, l'espace de plus grand diamètre (151b) étant sur la partie inférieure du corps (151), l'espace entier collaborant avec le piston-tiroir (152) guidé par rapport au corps (151) par deux lots de quatre vis à bout cylindrique (156) répartis sur le corps. Dans chacun des lots, les vis à bout cylindrique (156) placées deux à deux de manière diamétralement opposée par rapport au corps (151) traversent le corps (151) dans des taraudages (157) formés sur le corps (151). Chacune des vis à bout cylindrique (156) a son corps qui traverse un orifice oblong (158) formé sur toute l'épaisseur du piston-tiroir (152) longitudinalement par rapport à l'axe du piston-tiroir (152), les vis à bout cylindrique (156) bloquent ainsi le piston-tiroir (152) en rotation par rapport au corps (151) mais autorisent un coulissement du piston-tiroir (152) par rapport au corps (151) du fait de la course des vis à bout cylindrique (156) dans les orifices oblongs (158). Les vis à bout cylindrique (156) et les orifices oblongs (158) sont placés respectivement sur le corps (151) et sur le piston-tiroir (152) de manière à définir deux positions distinctes du piston-tiroir (152) par rapport au corps(151). Le piston tiroir (152) prend deux positions à l'intérieur du corps (151) correspondant aux fins de course des vis à bout cylindrique (156) dans les orifices oblongs (158). Dans une première position dite basse, la partie de plus grand diamètre (152b) du piston-tiroir (152) fait en partie saillie de la face inférieure du corps (151) et dans une deuxième position dite haute, le piston-tiroir (152) est enfoncé dans le corps (151) et collabore parfaitement avec l'espace intérieur des cylindres (151 a, 151b). L'extrémité des vis (156) vient faire saillie dans le perçage formé longitudinalement sur le piston-tiroir de manière à limiter les frottements lorsque l'équipage mobile (15) coulisse le long de la tige (13).

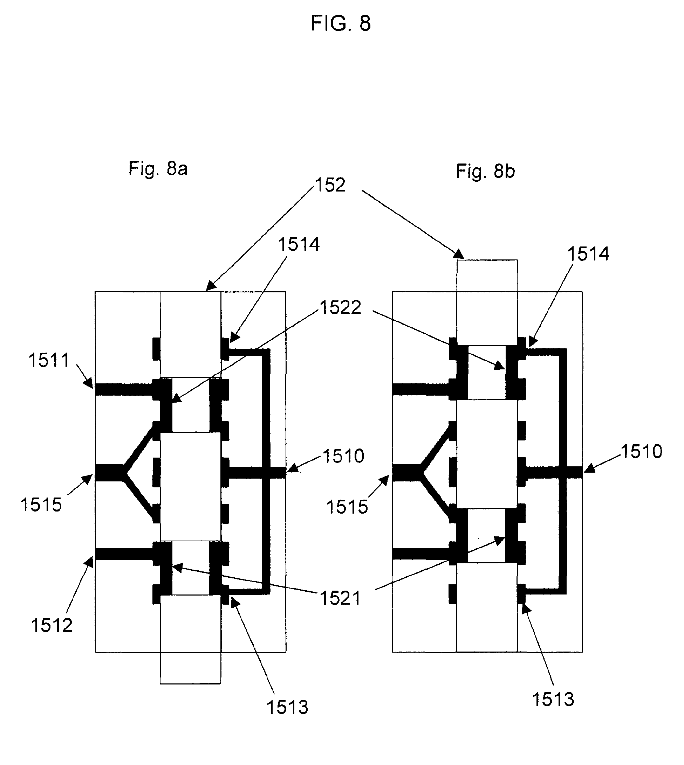

Deux réseaux de conduits sont formés à l'intérieur de la paroi du corps (151). Ces réseaux sont tous les deux reliés à la même alimentation (1510) en liquide hydraulique. Un réseau alimente le vérin (19) par l'intermédiaire d'un conduit (1511) dans sa chambre inférieure et l'autre réseau alimente le vérin (19) par l'intermédiaire d'un deuxième conduit (1512) dans sa chambre supérieure. Suivant la position du piston-tiroir (152) à l'intérieur du corps (151), le vérin (19) pourra entraîner l'équipage mobile (15) à tiroir hydraulique le long de la tige (13) dans le sens de la montée ou de la descente. Pour cela, deux gorges (1521, 1522) sont formées sur le périmètre de la paroi extérieure de plus petit diamètre (152a) du piston-tiroir (152). La position du piston-tiroir (152) détermine le réseau d'alimentation du vérin (19) en liquide hydraulique. Lorsque le piston-tiroir (152) est en position basse comme visualisé sur la fig. 8a, la gorge (1521) est en vis à vis d'un conduit (1513) d'un réseau et l'autre conduit (1514) du second réseau est bouché par la paroi externe du piston (152). Le liquide hydraulique passe par le conduit d'alimentation (1510) puis circule dans la gorge (1521) située en vis à vis du conduit (1513) puis alimente le vérin (19) par le conduit (1512). Le liquide remplit la chambre supérieure du vérin (19) et pousse le piston du vérin (19) vers le bas provoquant le coulissement vers le bas le long de la potence du chariot (18) mobile solidaire de l'équipage mobile (15). Ainsi le liquide va alimenter le vérin en ne traversant qu'un seul réseau. Lorsque le piston-tiroir (152) est en position haute dans le corps (151) comme représenté fig. 8b, le liquide hydraulique emprunte l'autre réseau. Il traverse la deuxième gorge (1522) par l'intermédiaire du conduit (1514) et alimente le vérin (19) par la sortie (1511) joignant la chambre inférieure du vérin (19). Le liquide pousse le piston du vérin (19) vers le haut entraînant dans le même sens le chariot (18) mobile solidaire de l'équipage mobile (15). Lorsque le vérin (19) est alimenté dans une de ses chambres, le piston du vérin (19) est en mouvement et le liquide contenu dans l'autre chambre est évacué par un autre conduit (1515).

Le piston-tiroir (152) actionne dans ses déplacements par rapport au corps (151) des billes (159) logées dans une gorge (160) usinée transversalement par rapport à l'axe du piston (152) sur le périmètre de la paroi externe de la partie de plus grand diamètre du piston (152b). Chacune des billes (159) fait saillie de la gorge (160) dans un orifice (161) formé sur le corps (151). La bille (159) est bloquée par les parois internes de la gorge (160) et la paroi interne de l'orifice (161) et vers l'extérieur de l'orifice (161) par une bague (154) susceptible de coulisser le long du corps (151).

Sur la base de la partie supérieure du piston (152), se dresse un fût cylindrique (162). Ce fût cylindrique (162) est destiné à venir en butée sur la butée (17) située au-dessus du système à tiroir hydraulique (15) et fixée sur la tige (13). Cette butée (17) est réglable en hauteur le long de la tige (13) graduée et est constituée d'un corps (171) percé en son centre pour laisser passer la (13) tige et de deux vis (172) vissées dans le corps (171) de manière symétriquement opposée à l'axe de la tige (13) et perpendiculairement à l'axe de la tige (13). Ces vis (172) viennent serrer chacune une bille (173) contre la tige (13). La butée (17) est donc débrayable ce qui veut dire que dans le cas d'un choc important du fût cylindrique (162) elle sera susceptible de se déplacer le long de la tige (13). Cette butée (17) est liée à un ressort (21), ce ressort (21) étant fixé à une autre butée (20) fixe formée au sommet de la tige (13).

L'équipage mobile (15) à tiroir hydraulique est surmonté d'un cardan (153) constitué d'une partie basse (1531), d'une chemise (1532) et d'une partie haute (1533). Ce cardan est destiné à guider la tige (13) et à lui permettre de dévier au cas où la tige d'enfoncement (11) viendrait se dévier en heurtant un matériau dur dans le sol (2). Des joints d'étanchéité (163) sont placés entre la chemise (1532) du cardan (153) et le corps (151) et entre le piston (152) et le corps (151) pour éviter un écoulement de liquide hydraulique.

La masse (14) est creusée sur sa partie supérieure d'un trou (142) circulaire formant un épaulement autour du perçage (141) prévu pour le coulissement de la masse (14). Les bords du trou (143) font saillie vers l'axe de la tige (13).

Le dispositif fonctionne de la manière suivante.

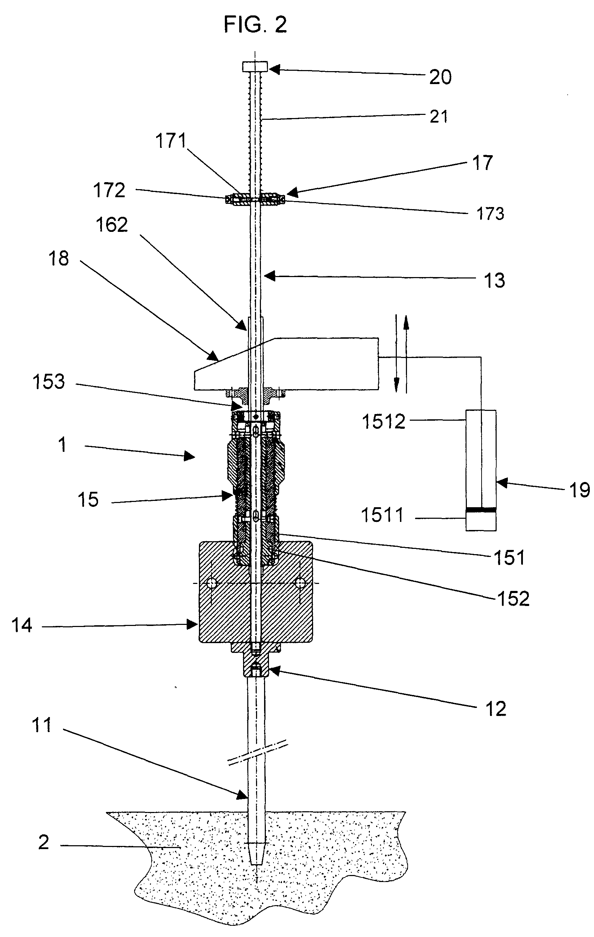

Sur les figures 1 et 2 :

L'équipage mobile (15) à tiroir hydraulique entraîné par le vérin (19) coulisse le long de la tige (13) dans le sens de la descente, le piston-tiroir (152) est dans sa première position dite basse représentée fig. 8a. La masse (14) est alors posée sur l'enclume (12). Les bords (143) du trou (142) de la masse laissent pénétrer dans le trou (142) les parties inférieures du corps (151b) et du piston (152b), la bague (154) vient en butée à l'extérieur du trou contre les bords (143) et coulisse le long du corps (151) entraînée par le déplacement de l'équipage mobile (15) contre l'action du ressort (155). Le retrait de la bague (154) libère les orifices (161) en face desquels les billes (159) sont logés dans la gorge (160). Lorsque la partie saillante du piston (152) arrive en butée au fond du trou (142), le piston (152) s'enfonce dans le corps (151) provoquant par une rampe (1601) usinée sur la gorge (160) la sortie des billes (159) en saillie des orifices (161). Lorsque l'équipage mobile (15) va remonter, les billes (159) vont venir en butée à l'intérieur du trou (142) sur les bords (143) en saillie, la masse (14), grâce à son poids, va ainsi être verrouillée sur l'équipage mobile (15) à tiroir hydraulique.

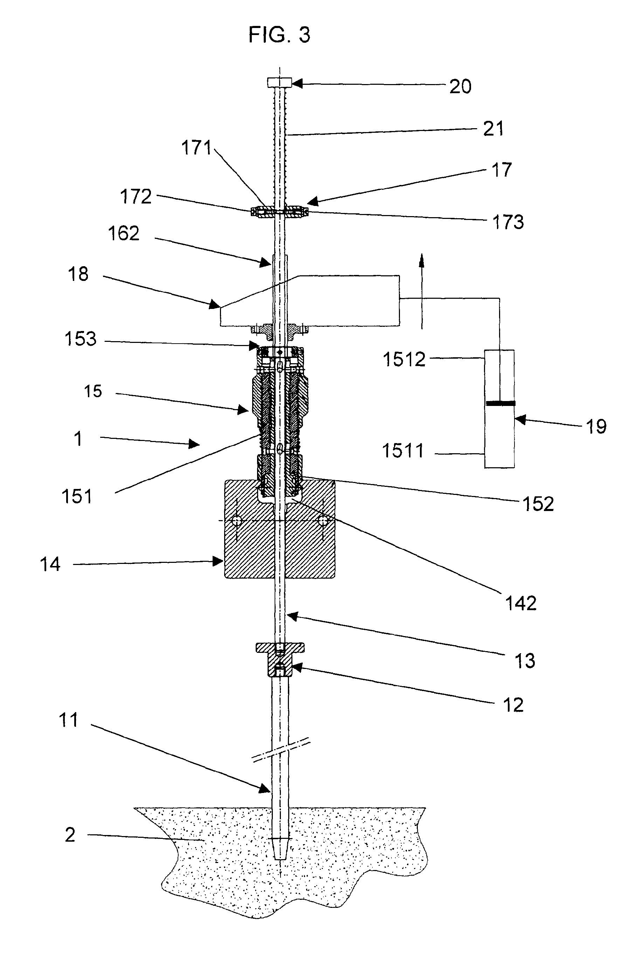

Sur la figure 3 :

Le piston-tiroir (152) est donc passé dans sa seconde position dite haute représentée fig. 8b, ce qui provoque le passage du liquide dans le second réseau d'alimentation signifiant le fonctionnement du vérin (19) dans l'autre sens pour remonter le chariot mobile (18) solidaire de l'équipage mobile (15) en compagnie de la masse (14).

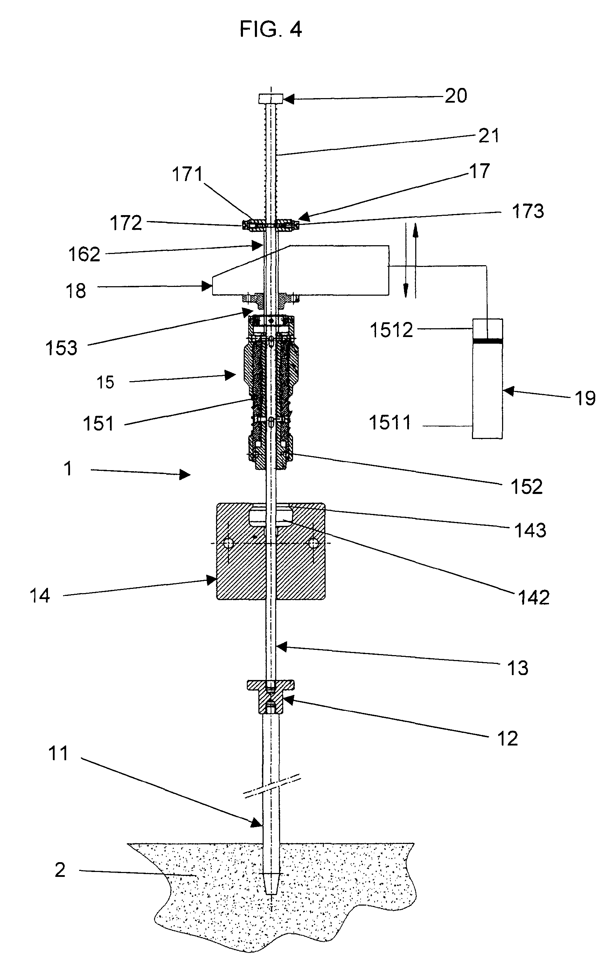

Sur la figure 4 :

Lorsque le fût cylindrique (162) dressé sur la face supérieure du piston (152) vient en butée contre la butée (17) débrayable, le piston-tiroir (152) est repoussé par la butée (17) agissant sur le fût (162) vers sa première position dite basse de la fig. 8a. Le déplacement relatif du piston-tiroir (152) vers le bas permet un retour des billes (159) dans la gorge (160) et donc un déverrouillage de la masse (14) qui peut alors glisser en chute libre le long de la tige (13) pour aller taper l'enclume (12) et ainsi enfoncer la tête (11) dans le sol (2) d'une certaine profondeur. La bague (154) poussée par son ressort (155) revient dans sa position basse initiale pour empêcher les billes (159) de sortir vers l'extérieur des orifices (161) et les maintenir dans la gorge (160)

Le piston-tiroir (152) est revenu dans sa première position et inverse le sens de déplacement du vérin (19) qui va alors faire redescendre l'équipage mobile (15) par l'intermédiaire du chariot mobile (18) pour aller de nouveau chercher la masse (14) sur l'enclume (12).

La masse (14) tombe très vite sur l'enclume (12) et un temps de réaction est nécessaire au système à tiroir hydraulique (15) pour aller chercher la masse (14). Il se peut que la tige d'enfoncement (11) s'enfonce d'une profondeur assez importante si le terrain est relativement meuble. Dans ce cas la butée (17) est susceptible de venir heurter le fût cylindrique (162). C'est pourquoi, la butée (17) est débrayable en cas de choc, elle va remonter le long de la tige (13). Grâce à son ressort (21) de poussée elle revient dans sa position initiale afin de ne pas modifier la hauteur de chute de la masse (14).

Ce kit (1) est adaptable sur n'importe quel système support comportant un mécanisme d'entraînement vers le haut et vers le bas par l'intermédiaire de raccords rapides. De plus ce kit (1) peut aussi bien servir pour de la pénétrométrie que pour du forage. La masse (14) est amovible et la butée (17) est réglable en hauteur le long de la tige (13) graduée.

Le but de l'invention est atteint par un kit de pénétrométrie (1) constitué d'une tige d'enfoncement (11) surmontée d'une enclume (12) sur laquelle vient taper en chute libre une masse (14) caractérisé en ce qu'au centre de la masse (14), sur toute son épaisseur est réalisé un perçage (141) par lequel passe une tige (13) rendue solidaire de l'enclume (12) qui se dresse perpendiculairement au plan constitué par la face supérieure de l'enclume (12), un équipage mobile (15) à tiroir hydraulique se déplace alternativement sur ladite tige (13) entre une position haute et une position basse, l'équipage mobile (15) comportant des moyens de libération de la masse (14) arrivée en position haute pour permettre sa chute libre et des moyens de solidarisation avec la masse (14) lors de son arrivée en position basse, un piston-tiroir (152) actionné par ces moyens de solidarisation et de libération inversant le sens de déplacement de l'équipage mobile (15).

Selon une autre particularité, un chariot (18) entraîné le long d'une potence parallèle à la tige (13) par un moteur ou un vérin (19) est fixé à l'équipage mobile (15).

Selon une autre particularité, l'équipage mobile (15) à tiroir hydraulique comprend un corps (151) ayant la forme d'un fût cylindrique creux ouvert sur ses faces inférieure et supérieure et à l'intérieur duquel un piston-tiroir (152) traversé longitudinalement par la tige (13) est susceptible de coulisser entre deux positions et un moyen de libération de la masse surmontant le piston-tiroir (152) et destiné à venir en butée sur la butée (17) fixée sur la partie supérieure de la tige.

Selon une autre particularité, le moyen de libération de la masse est un fût cylindrique (162) se dressant perpendiculairement à la face supérieure du piston-tiroir (152) et coulissant le long de la tige (13).

Selon une autre particularité, l'espace intérieur du corps (151) creux a la forme de deux fûts cylindriques (151a, 151b) fourreau de diamètres différents accolés, l'espace de plus grand diamètre (151b) étant sur la partie inférieure du corps et en ce que le piston-tiroir (152) a des dimensions telles que lorsqu'il est enfoncé dans le corps (151) il collabore parfaitement avec l'espace intérieur du corps (151).

Selon une autre particularité, la forme externe du corps (151) est cylindrique.

Selon une autre particularité, le piston-tiroir (152) est guidé en translation (151) par rapport au corps (151) par au moins une vis à bout cylindrique (156) traversant transversalement le corps (151) à travers un taraudage (157) formé sur l'épaisseur de celui-ci, le corps de la vis à bout cylindrique (156) traversant un orifice oblong (158) formé sur le piston-tiroir et son extrémité faisant saillie dans le perçage formé longitudinalement sur le piston-tiroir (152) pour le passage de la tige (13).

Selon une autre particularité, deux gorges (1521, 1522) sont usinées transversalement par rapport à l'axe du piston-tiroir (152) sur tout le périmètre de la paroi externe de plus petit diamètre (152a) du piston-tiroir (152) et que deux réseaux de conduits reliés tous les deux à une extrémité à une alimentation en liquide hydraulique (1510) et à l'autre extrémité en deux points différents à un moteur ou un vérin (19) sont formés à l'intérieur du corps (151), le piston (152) par sa position par rapport au corps (151) et par l'intermédiaire de ses gorges (1521, 1522) servant à sélectionner un réseau pour faire traverser le liquide entre l'alimentation (1510) et le moteur ou le vérin (19).

Selon une autre particularité, le liquide, selon le réseau emprunté, est destiné à commander le moteur ou le vérin (19) dans le sens de la montée ou de la descente de l'équipage mobile (15) à tiroir hydraulique.

Selon une autre particularité, le moyen de solidarisation de l'équipage mobile (15) sur la masse (14) est un système de verrouillage par billes du système (15) sur la masse (14), des billes (159) logées dans une gorge (160) usinée transversalement par rapport à l'axe du piston (152) et sur tout le périmètre de la paroi externe de la partie inférieure du piston, chacune des billes (159) faisant saillie de cette paroi dans un orifice (161) réalisé sur le corps (151) dans le prolongement de la gorge (160), la bille (159) étant bloquée par les parois internes de la gorge (160) et de l'orifice (161) et vers l'extérieur de l'orifice (161) par une bague (154) susceptible de coulisser le long du corps (151).

Selon une autre particularité, les billes (159) sont logées dans la partie de plus grand diamètre du piston-tiroir (152b).

Selon une autre particularité, dans sa première position le piston-tiroir (152) est bloqué par rapport au corps (151) par les billes (159), la partie de plus grand diamètre du piston-tiroir (152b) fait alors en partie saillie du côté de la face inférieure du corps (151) et dans sa deuxième position, le piston-tiroir (152) est totalement enfoncé dans le corps (151).

Selon une autre particularité, une extrémité d'un ressort (155) de rappel entourant le corps (151) est fixée en haut du corps (151) et en ce que l'autre extrémité est fixée à la bague (155), permettant à la bague (155) de coulisser le long du corps (155).

Selon une autre particularité, la masse (14) est creusée (142) sur sa partie supérieure autour du perçage (141) par lequel passe la tige (13) pour recevoir la partie inférieure de l'équipage mobile (15), les bords (143) du trou faisant saillie vers l'axe de la tige (13) de manière à ne laisser passer que le corps (151) et le piston-tiroir (152) et à servir de butée à la bague (154) qui va alors coulisser le long du corps (151) par l'intermédiaire de son ressort (155) lors de l'avancée de l'équipage (15) dans le trou (142) et lorsque le piston-tiroir (152) arrive en butée au fond du trou (142), le piston-tiroir (152) coulisse par rapport au corps (151), ce qui provoque l'extraction des billes (159) de la gorge (160) qui vont alors faire saillie du corps (151) pour venir bloquer la masse (14) sur l'équipage mobile (15).

Selon une autre particularité, la paroi latérale inférieure de la gorge (160) a la forme d'une rampe (1601) de manière à faciliter l'extraction des billes (159).

Selon une autre particularité, la butée (17) fixée sur la partie supérieure de la tige est débrayable et fixée à un ressort de rappel (21) fixé en haut de la tige (13).

Selon une autre particularité, la butée (17) de débrayage est constituée d'un corps (171) traversé symétriquement par la tige (13) et d'au moins deux billes (173) logées dans le corps (171), chacune des billes serrant la tige (13) par l'intermédiaire d'une vis (172) vissée dans le corps (171) de la butée (17) perpendiculairement à l'axe de la tige (13).

Selon une autre particularité, la butée (17) fixée sur la tige (13) est réglable en hauteur le long de la tige (13) pour obtenir des hauteurs de chute différentes.

Selon une autre particularité, un cardan (153) placé au-dessus de l'équipage mobile (15) à tiroir hydraulique permet de guider la tige (13).

Selon une autre particularité, la tige d'enfoncement (11) est vissée sur la face inférieure de l'enclume (12).

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Kit de pénétrométrie (1) constitué d'une tige d'enfoncement (11) surmontée d'une enclume (12) sur laquelle vient taper en chute libre une masse (14) **caractérisé en ce qu'**au centre de la masse (14), sur toute son épaisseur est réalisé un perçage (141) par lequel passe une tige (13) rendue solidaire de l'enclume (12) qui se dresse perpendiculairement au plan constitué par la face supérieure de l'enclume (12), un équipage mobile (15) à tiroir hydraulique se déplace alternativement sur ladite tige (13) entre une position haute et une position basse, l'équipage mobile (15) comportant des moyens de libération de la masse (14) arrivée en position haute pour permettre sa chute libre et des moyens de solidarisation avec la masse (14) lors de son arrivée en position basse, un piston-tiroir (152) actionné par ces moyens de solidarisation et de libération inversant le sens de déplacement de l'équipage mobile (15).

2. Kit de pénétrométrie selon la revendication 1 **caractérisé en ce qu'**un chariot (18) entraîné le long d'une potence parallèle à la tige (13) par un moteur ou un vérin (19) est fixé à l'équipage mobile (15).

3. Kit de pénétrométrie (1) selon la revendication 1 ou 2 **caractérisé en ce que** l'équipage mobile (15) à tiroir hydraulique comprend un corps (151) ayant la forme d'un fût cylindrique creux ouvert sur ses faces inférieure et supérieure et à l'intérieur duquel un piston-tiroir (152) traversé longitudinalement par la tige (13) est susceptible de coulisser entre deux positions et un moyen de libération de la masse surmontant le piston-tiroir (152) et destiné à venir en butée sur la butée (17) fixée sur la partie supérieure de la tige.

4. Kit de pénétrométrie (1) selon la revendication 1 ou 3 **caractérisé en ce que** le moyen de libération de la masse est un fût cylindrique (162) se dressant perpendiculairement à la face supérieure du piston-tiroir (152) et coulissant le long de la tige (13).

5. Kit de pénétrométrie (1) selon la revendication 3 **caractérisé en ce que** l'espace intérieur du corps (151) creux a la forme de deux fûts cylindriques (151a, 151b) fourreau de diamètres différents accolés, l'espace de plus grand diamètre (151b) étant sur la partie inférieure du corps et **en ce que** le piston-tiroir (152) a des dimensions telles que lorsqu'il est enfoncé dans le corps (151) il collabore parfaitement avec l'espace intérieur du corps (151).

6. Kit pénétrométrie (1) selon la revendication 3 ou 5 **caractérisé en ce que** la forme externe du corps (151) est cylindrique.

7. Kit de pénétrométrie (1) selon la revendication 3 ou 5 **caractérisé en ce que** le piston-tiroir (152) est guidé en translation (151) par rapport au corps (151) par au moins une vis à bout cylindrique (156) traversant transversalement le corps (151) à travers un taraudage (157) formé sur l'épaisseur de celui-ci, le corps de la vis à bout cylindrique (156) traversant un orifice oblong (158) formé sur le piston-tiroir et son extrémité faisant saillie dans le perçage formé longitudinalement sur le piston-tiroir (152) pour le passage de la tige (13).

8. Kit de pénétrométrie selon la revendication 5 **caractérisé en ce que** deux gorges (1521, 1522) sont usinées transversalement par rapport à l'axe du piston-tiroir (152) sur tout le périmètre de la paroi externe de plus petit diamètre (152a) du piston-tiroir (152) et que deux réseaux de conduits reliés tous les deux à une extrémité à une alimentation en liquide hydraulique (1510) et à l'autre extrémité en deux points différents à un moteur ou un vérin (19) sont formés à l'intérieur du corps (151), le piston (152) par sa position par rapport au corps (151) et par l'intermédiaire de ses gorges (1521, 1522) servant à sélectionner un réseau pour faire traverser le liquide entre l'alimentation (1510) et le moteur ou le vérin (19).

9. Kit de pénétrométrie (1) selon la revendication 8 **caractérisé en ce que** le liquide selon le réseau emprunté est destiné à commander le moteur ou le vérin (19) dans le sens de la montée ou de la descente de l'équipage mobile (15) à tiroir hydraulique.

10. Kit de pénétrométrie (1) selon la revendication 1 **caractérisé en ce que** le moyen de solidarisation de l'équipage mobile (15) sur la masse (14) est un système de verrouillage par billes du système (15) sur la masse (14), des billes (159) logées dans une gorge (160) usinée transversalement par rapport à l'axe du piston (152) et sur tout le périmètre de la paroi externe de la partie inférieure du piston, chacune des billes (159) faisant saillie de cette paroi dans un orifice (161) réalisé sur le corps (151) dans le prolongement de la gorge (160), la bille (159) étant bloquée par les parois internes de la gorge (160) et de l'orifice (161) et vers l'extérieur de l'orifice (161) par une bague (154) susceptible de coulisser le long du corps (151).

11. Kit de pénétrométrie (1) selon la revendication 10 **caractérisé en ce que** les billes (159) sont logées dans la partie de plus grand diamètre du piston-tiroir (152b).

12. Kit de pénétrométrie (1) selon la revendication 9 ou 10 **caractérisé en ce que** dans sa première position le piston-tiroir (152) est bloqué par rapport au corps (151) par les billes (159), la partie de plus grand diamètre du piston-tiroir (152b) fait alors en partie saillie du côté de la face inférieure du corps (151) et dans sa deuxième position, le piston-tiroir (152) est totalement enfoncé dans le corps (151).

13. Kit de pénétrométrie (1) selon la revendication 10 **caractérisé en ce qu'**une extrémité d'un ressort (155) de rappel entourant le corps (151) est fixée en haut du corps (151) et **en ce que** l'autre extrémité est fixée à la bague (155), permettant à la bague (155) de coulisser le long du corps (155).

14. Kit de pénétrométrie (1) selon la revendication **caractérisé en ce que** la masse (14) est creusée (142) sur sa partie supérieure autour du perçage (141) par lequel passe la tige (13) pour recevoir la partie inférieure de l'équipage mobile (15), les bords (143) du trou faisant saillie vers l'axe de la tige (13) de manière à ne laisser passer que le corps (151) et le piston-tiroir (152) et à servir de butée à la bague (154) qui va alors coulisser le long du corps (151) par l'intermédiaire de son ressort (155) lors de l'avancée de l'équipage (15) dans le trou (142) et lorsque le piston-tiroir (152) arrive en butée au fond du trou (142), le piston-tiroir (152) coulisse par rapport au corps (151), ce qui provoque l'extraction des billes (159) de la gorge (160) qui vont alors faire saillie du corps (151) pour venir bloquer la masse (14) sur l'équipage mobile (15).

15. Kit de pénétrométrie (1) selon la revendication 10 ou 14 **caractérisé en ce que** la paroi latérale inférieure de la gorge (160) a la forme d'une rampe (1601) de manière à faciliter l'extraction des billes (159).

16. Kit de pénétrométrie (1) selon la revendication 1 **caractérisé en ce que** la butée (17) fixée sur la partie supérieure de la tige est débrayable et fixée à un ressort de rappel (21) fixé en haut de la tige (13).

17. Kit de pénétrométrie selon la revendication 16 **caractérisé en ce que** la butée (17) de débrayage est constituée d'un corps (171) traversé symétriquement par la tige (13) et d'au moins deux billes (173) logées dans le corps (171), chacune des billes serrant la tige (13) par l'intermédiaire d'une vis (172) vissée dans le corps (171) de la butée (17) perpendiculairement à l'axe de la tige (13).

18. Kit de pénétrométrie (1) selon la revendication 16 ou 17 **caractérisé en ce que** la butée (17) fixée sur la tige (13) est réglable en hauteur le long de la tige (13) pour obtenir des hauteurs de chute différentes.

19. Kit de pénétrométrie (1) selon la revendication 1 **caractérisé en ce qu'**un cardan (153) placé au-dessus de l'équipage mobile (15) à tiroir hydraulique permet de guider la tige (13).

20. Kit de pénétrométrie (1) selon la revendication 1 **caractérisé en ce que** la tige d'enfoncement (11) est vissée sur la face inférieure de l'enclume (12).
